# FASCICULE DE BREVET EUROPEEN

(11) **EP 3 454 861 B1**
(45) Date de publication et mention de la délivrance du brevet: **03.09.2025**
(21) Numéro de dépôt: 17727812.4
(22) Date de dépôt: 11.05.2017
(51) Int. Cl.: A61K 31/517, A61K 31/536, A61P 25/28

(54) **DERIVES DE LA BENZOXAZINE OU DE LA QUINAZOLINE POUR LEUR UTILISATION DANS LE TRAITEMENT DE LA MALADIE D'ALZHEIMER**
BENZOXAZIN- ODER CHINAZOLINDERIVATE ZUR VERWENDUNG BEI DER BEHANDLUNG VON MORBUS ALZHEIMER
BENZOXAZINE OR QUINAZOLINE DERIVATIVES FOR USE IN THE TREATMENT OF ALZHEIMER'S DISEASE

(30) Priorité: 11.05.2016 FR 1670222
(43) Date de publication de la demande: 20.03.2019
(73) Titulaire: Biocodex, 94250 Gentilly (FR)
(72) Inventeur: RIBAN, Véronique, 60200 Compiègne (FR); VERLEYE, Marc, 60190 Remy (FR); LE GUERN, Marie-Emanuelle, 60200 Compiègne (FR)
(74) Mandataire: Vial, Lionel
(86) Numéro de dépôt international: PCT/EP2017/061371
(87) Numéro de publication internationale: WO 2017/194692

(56) Documents cités:
- EP-A1- 1 745 786
- EP-A2- 2 116 247
- KIM HYO GEUN ET AL: "Donepezil inhibits the amyloid-beta oligomer-induced microglial activationin vitroandin vivo", NEUROTOXICOLOGY, TOX PRESS, RADFIELD, AR, IN, vol. 40, 1 November 2013 (2013-11-01), pages 23 - 32, XP028829480, ISSN: 0161-813X, DOI: 10.1016/J.NEURO.2013.10.004

## Description

### Domaine de l'invention

La présente invention concerne des composés et des compositions pharmaceutiques utiles dans la prévention ou le traitement de la maladie d'Alzheimer, par inhibition de l'action du peptide β-amyloïde.

### Arrière-plan technique

La maladie d'Alzheimer est la cause principale de dépendance lourde chez les personnes âgées. En 2015, on estime que 47,5 millions de personnes sont atteintes de la maladie à travers le monde. La prévalence de cette maladie est ainsi de 5% après 65 ans et croît de façon exponentielle avec l'âge (25% des plus de 80 ans sont touchés) mais elle peut également survenir beaucoup plus tôt.

La maladie d'Alzheimer est une maladie neurodégénérative, c'est-à-dire qu'elle entraine une perte progressive et irréversible des neurones. Cette perte entraine une altération des facultés cognitives, telle que la mémoire, le langage, le raisonnement, ainsi qu'une disparition des capacités d'orientation dans le temps et dans l'espace.

La maladie d'Alzheimer semble être notamment la conséquence d'une accumulation de plaques amyloïdes, provenant du dépôt, en dehors des neurones, du peptide β-amyloïde (ou amyloïde β, Aβ). Ces plaques entrainent un dysfonctionnement des neurones environnants, puis la mort neuronale. Le peptide β-amyloïde comprend 36 à 43 acides aminés et provient du clivage enzymatique anormal de la protéine précurseur de l'amyloïde (APP), par la β-secrétase et la γ-secrétase. Ce peptide est insoluble et peu dégradé. Ce processus débute généralement au niveau de l'hippocampe et s'étend progressivement aux différentes zones du cortex cérébral.

A l'heure actuelle aucun traitement curatif ne permet de guérir la maladie d'Alzheimer. En effet, les médicaments actuellement autorisés dans ce cadre, dont notamment le donépézil (Rogers et al. (1998) Arch Intern Med. 158:1021-3), un anti-cholinestérasique qui inhibe l'activation microgliale, responsable de la mort neuronale, induite par les formes oligomériques solubles du peptide β-amyloïde (Kim et al. (2014) Neurotoxicology 40:23-32), permettent au mieux de ralentir la progression symptomatique de la maladie mais ils n'empêchent pas la dégénérescence et la mort neuronale.

Il reste donc nécessaire de trouver des alternatives à ces composés, permettant notamment de s'opposer aux mécanismes pathologiques de cette maladie, en plus de traiter les symptômes.

L'étifoxine, ou chlorhydrate de 6-chloro-2-éthylamino-4-méthyl-4-phényl-4H-[3,1]benzoxazine appartient à la famille des aminobenzoxazines. Elle favorise la transmission gabaergique en se liant sur un site proche du canal chlore couplé au récepteur GABA_{A} et est actuellement utilisée comme anxiolytique. Peu de manifestations indésirables consécutives à son utilisation sont répertoriées.

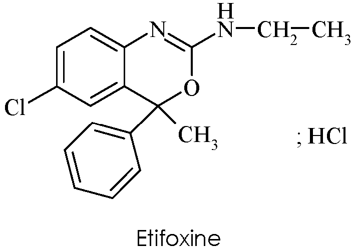

La synthèse de ce composé est notamment décrite dans le brevet français n° 1 571 287. Par ailleurs, plusieurs métabolites actifs de l'étifoxine ont été décrits, tels que la déséthyl-étifoxine ou 2-amino-6-chloro-4-méthyl-4-phényl-4H-[3,1]benzoxazine, la 6-chloro-4-(4-hydroxyphényl)-4-méthyl-3,4-dihydro-1H-quinazolin-2-one ou la 6-chloro-3-éthyl-7-hydroxy-4-méthyl-4-phényl-3,4-dihydro-1H-quinazolin-2-one.

La demande de brevet EP1745786 décrit l'utilisation de l'étifoxine pour la préparation d'un médicament à activité neuroprotectrice destiné à la prévention ou au traitement des détériorations neuronales. La demande de brevet EP2116247 concerne utilisation de l'étifoxine pour la préparation d'un médicament destiné à la prévention ou au traitement des troubles mnésiques.

### Résumé de l'invention

La présente invention découle de la mise en évidence inattendue que l'étifoxine permettait de traiter les troubles cognitifs dans un modèle murin de la maladie d'Alzheimer. Par ailleurs, les inventeurs ont également montré que l'étifoxine s'opposait aux effets pathologiques du peptide β-amyloïde.

La présente invention est telle que définie dans les revendications 1 à 13.

Ainsi, la présente invention concerne un composé de formule (I) suivante : 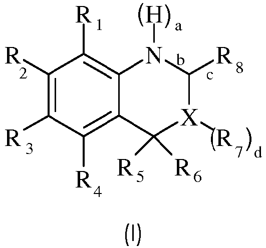 dans laquelle :
- a représente 0 ou 1 ;
- b représente une simple liaison ou une double liaison ;
- c représente une simple liaison ou une double liaison ;
- d représente 0 ou 1 ;
- X représente un atome d'oxygène ou d'azote, sous réserve que lorsque X représente un atome d'oxygène alors d vaut 0 et que lorsque X représente un atome d'azote alors d vaut 1;
- R₁, R₂, R₃, et R₄, identiques ou différents, représentent un atome d'hydrogène, un atome d'halogène, notamment choisi parmi F, Cl, Br, ou I, un groupe hydroxyle, ou un groupe alcoxyle de 1 ou 2 atomes de carbone ;
- R₅ et R₆, identiques ou différents, représentent un atome d'hydrogène, un groupe alkyle ou cycloalkyle de 1 à 6 atomes de carbone, ou un groupe aryle de 6 atomes de carbone dont le noyau aromatique est éventuellement substitué par un ou plusieurs atomes d'halogène ou un ou plusieurs groupes hydroxyle, alcoxyle de 1 ou 2 atomes de carbone, trifluorométhyle ou nitro ;
- R₇ représente un atome d'hydrogène, un groupe hydroxyle, ou un groupe alkyle ou hydroxyalkyle de 1 à 3 atomes de carbone ;
- R₈ représente un atome d'oxygène ou un groupe -NR₉R₁₀, R₉ et R₁₀, identiques ou différents, représentant un atome d'hydrogène, un groupe hydroxyle, ou un groupe alkyle ou hydroxyalkyle de 1 à 3 atomes de carbone, sous réserve que lorsque R₈ représente un atome d'oxygène alors a vaut 1, b représente une simple liaison et c représente une double liaison et que lorsque R₈ représente un groupe -NR₉R₁₀ alors a vaut 0, b représente une double liaison et c représente une simple liaison ;
ou sel pharmaceutiquement acceptable de celui-ci,
pour son utilisation dans la prévention ou le traitement de la maladie d'Alzheimer chez un individu, par inhibition de l'action du peptide β-amyloïde.

Dans un mode de réalisation particulier de la description, le composé ou le sel pharmaceutiquement acceptable de celui-ci pour son utilisation telle que définie ci-dessus est en combinaison avec au moins un autre composé utile pour la prévention ou le traitement de la maladie d'Alzheimer.

La présente invention concerne également une composition pharmaceutique ou un médicament, comprenant à titre de substance active, au moins un composé de formule (I) tel que défini ci-dessus, ou un sel pharmaceutiquement acceptable de celui-ci, éventuellement en association avec un véhicule pharmaceutiquement acceptable, pour son utilisation dans la prévention ou le traitement de la maladie d'Alzheimer chez un individu, par inhibition de l'action du peptide β-amyloïde.

Dans un mode de réalisation particulier de l'invention, la composition pharmaceutique pour son utilisation telle que définie ci-dessus comprend au moins un autre composé utile pour la prévention ou le traitement de la maladie d'Alzheimer.

La présente description concerne également une composition pharmaceutique, comprenant à titre de substances actives, au moins un composé de formule (I) tel que défini ci-dessus, ou un sel pharmaceutiquement acceptable de celui-ci, ainsi qu'au moins un autre composé utile pour la prévention ou le traitement de la maladie d'Alzheimer, éventuellement en association avec un véhicule pharmaceutiquement acceptable.

La présente invention concerne également des produits contenant :
- au moins un composé de formule (I) tel que défini ci-dessus, ou un sel pharmaceutiquement acceptable de celui-ci, et
- au moins un autre composé utile pour la prévention ou le traitement de la maladie d'Alzheimer,
comme produit de combinaison pour une utilisation, notamment simultanée, séparée ou étalée dans le temps, pour la prévention ou le traitement de la maladie d'Alzheimer chez un individu, par inhibition de l'action du peptide β-amyloïde.

La présente invention concerne également une méthode pour prévenir ou traiter la maladie d'Alzheimer chez un individu, par inhibition de l'action du peptide β-amyloïde, comprenant l'administration à l'individu d'une quantité efficace d'un composé de formule (I) tel que défini ci-dessus, ou d'un sel pharmaceutiquement acceptable de celui-ci.

Dans un mode de réalisation particulier de l'invention, la méthode telle que définie ci-dessus comprend également l'administration d'au moins un autre composé utile pour la prévention ou le traitement de la maladie d'Alzheimer à l'individu.

La présente invention concerne également l'utilisation d'un composé de formule (I) tel que défini ci-dessus, ou d'un sel pharmaceutiquement acceptable de celui-ci, pour la préparation d'un médicament destiné à la prévention ou au traitement de la maladie d'Alzheimer chez un individu, par inhibition de l'action du peptide β-amyloïde.

Dans un mode de réalisation particulier de l'invention, le médicament tel que défini ci-dessus comprend au moins un autre composé utile pour la prévention ou le traitement de la maladie d'Alzheimer.

### Description détaillée de l'invention

### Maladie d'Alzheimer

La maladie d'Alzheimer est bien connue de l'homme du métier. Elle est notamment définie par la classe G30 dans la 10^{ème} révision de la classification internationale des maladies (ICD - 10) version 2016 établie par l'Organisation Mondiale de la Santé. Par ailleurs, la maladie d'Alzheimer est définie par les critères diagnostiques suivants dans la cinquième édition du Manuel diagnostique et statistique des troubles mentaux (DSM-5) (2013) American Psychiatric Association, pages 611-614 :
A. Les critères du trouble neurocognitif majeur (démence) ou du trouble neurocognitif léger sont rencontrés.
B. Début insidieux et progression graduelle d'une déficience dans un ou plusieurs domaines cognitifs (pour le trouble neurocognitif majeur, au moins deux domaines doivent être déficients).
C. Les critères suivants pour la maladie d'Alzheimer probable ou possible sont rencontrés :
   ***Pour le trouble neurocognitif majeur** :*
   La **maladie d'Alzheimer probable** est diagnostiquée si une des conditions suivantes est présente ; autrement, la **maladie d'Alzheimer possible** devrait être diagnostiquée.
      1. Preuve d'une mutation génétique responsable de la maladie d'Alzheimer à partir de l'histoire de la famille ou d'un test génétique.
      2. Les trois éléments suivants sont présents :
         a. Preuve claire de déclin de la mémoire et de l'apprentissage et d'au moins un autre domaine cognitif (basée sur l'histoire détaillée ou une série de tests neuropsychologiques).
         b. Déclin progressif régulier de l'état cognitif, sans plateaux prolongés.
         c. Aucune preuve d'étiologie mixte (c'est-à-dire absence d'autres maladies neurodégénérative ou cérébrovasculaire, ou d'une autre maladie neurologique, mentale ou systémique ou d'un état susceptible de contribuer au déclin cognitif).
   ***Pour le trouble neurocognitif* léger** :
   La **maladie d'Alzheimer probable** est diagnostiquée s'il y a une preuve d'une mutation génétique responsable de la maladie d'Alzheimer provenant de tests génétiques ou des antécédents familiaux.
   La **maladie d'Alzheimer possible** est diagnostiquée s'il n'y a pas de preuve d'une mutation génétique responsable de la maladie d'Alzheimer provenant de tests génétiques ou de l'histoire de la famille, et les trois des éléments suivants sont présents :
      3. Preuve claire de déclin de la mémoire et de l'apprentissage.
      4. Déclin progressif régulier de l'état cognitif, sans plateaux prolongés.
      5. Aucune évidence d'étiologie mixte (c'est-à-dire, absence d'autres maladies neurodégénérative ou cérébrovasculaire, ou d'une autre maladie neurologique, ou systémique ou d'un état susceptible de contribuer au déclin cognitif).
D. La perturbation n'est pas mieux expliquée par une maladie cérébrovasculaire, une autre maladie neurodégénérative, les effets d'une substance ou d'un autre trouble mental, neurologique ou systémique.
   La maladie d'Alzheimer prévenue ou traitée selon l'invention peut notamment être la maladie d'Alzheimer à un stade précoce, la maladie d'Alzheimer à un stade intermédiaire, la maladie d'Alzheimer à un stade avancé, la maladie d'Alzheimer préclinique, une démence due à la maladie d'Alzheimer, un trouble cognitif léger dû à la maladie d'Alzheimer, un trouble neurocognitif léger dû à la maladie d'Alzheimer, un trouble neurocognitif majeur dû à la maladie d'Alzheimer, la maladie d'Alzheimer probable, ou la maladie d'Alzheimer possible.

De préférence, la prévention ou le traitement de la maladie d'Alzheimer selon l'invention désigne, la prévention ou le traitement d'au moins un trouble cognitif ou neurocognitif dû à la maladie d'Alzheimer, notamment sélectionné dans le groupe constitué d'un trouble de la mémoire ou d'un trouble de l'apprentissage.

La description concerne également la prévention ou le traitement des symptômes de la maladie d'Alzheimer par inhibition de l'action du peptide β-amyloïde.

Par ailleurs, selon l'invention la maladie d'Alzheimer est prévenue ou traitée par inhibition de l'action, ou des effets, en particulier pathologique(s) ou délétère(s), du peptide β-amyloïde. Comme on l'entend ici, le peptide β-amyloïde (ou peptide amyloïde β) se réfère à tout peptide, comprenant notamment de 36 à 43 acides aminés, résultant du clivage de la protéine précurseur de l'amyloïde (APP) par la β-secrétase et la γ-secrétase. De préférence, le peptide β-amyloïde selon l'invention désigne le peptide Aβ40 (également nommé Aβ₁₋₄₀) et/ou le peptide Aβ42 (également nommé Aβ₁₋₄₂). De préférence, l'action, ou les effets, en particulier pathologique(s) ou délétère(s), du peptide β-amyloïde selon l'invention est (sont) sélectionné(s) dans le groupe constitué de l'intoxication cellulaire, notamment neuronale, du stress oxydatif, notamment neuronal, de l'hyper-phosphorylation de la protéine tau, notamment neuronale, de la stimulation de l'expression, notamment au niveau de l'hippocampe, de facteurs pro-apoptotiques, notamment Bax et la Caspase-3, et de la stimulation de l'expression, notamment au niveau de l'hippocampe, de la protéine GFAP *(Glial Fibrillary Acidic Protein).*

### Individu

L'individu au sens de l'invention est de préférence un être humain.

Dans un mode de réalisation préférentiel de l'invention l'individu selon l'invention est âgé de 65 ans ou plus. Dans un mode de réalisation préférentiel alternatif de l'invention, l'individu selon l'invention est âgé de moins de 65 ans.

L'individu selon l'invention peut présenter un ou plusieurs symptômes de démence ou être atteint de démence.

L'individu selon l'invention peut également ne pas être atteint de démence. En particulier, l'individu selon l'invention peut être atteint de troubles cognitifs, notamment de troubles cognitifs légers, correspondant à la dénomination anglo-saxonne de Mild Cognitive Impairement (MCI), bien connue de l'homme du métier et notamment définie par Petersen et al. (1999) Arch Neurol 56:303-308. Un individu est généralement défini comme présentant un MCl en cas de plainte subjective associée à une évidence objective d'un déficit de la performance mnésique avec épargne du fonctionnement cognitif et intellectuel global et intégrité des activités de la vie de tous les jours. De préférence, un individu présentant un MCl selon l'invention a un score au test Mini Mental State Examination (MMSE), notamment dans la version consensuelle du Groupe de Réflexion sur les Evaluations Cognitives (GRECO), supérieur au score correspondant au 5ème centile, en fonction de son âge et de son niveau socio-culturel.

Par ailleurs, l'individu selon l'invention peut également ne pas présenter de troubles cognitifs.

### Composé de formule (I)

La synthèse des composés de formule (I) définie ci-dessus peut aisément être mise en œuvre à partir des enseignements du brevet français n° 1 571 287.

Les sels pharmaceutiquement acceptables selon l'invention apparaîtront de manière évidente pour l'homme du métier. En particulier les sels de chlorhydrate des composés de formule (I) selon l'invention sont préférés.

Comme on l'entend ici, la formule (I) définie ci-dessus englobe notamment les formules des composés de formule (I) optiquement actifs, tels que les énantiomères représentés par les formules suivantes (lorsque R₅ et R₆ sont différents) : 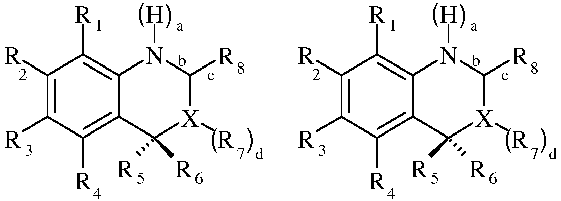 ou leurs mélanges, en particulier leur mélange racémique.

Dans un mode de réalisation préférentiel de l'invention, dans la formule (I) définie ci-dessus, R₅ et R₆, identiques ou différents, représentent un groupe alkyle ou cycloalkyle de 1 à 6 atomes de carbone, ou un groupe aryle de 6 atomes de carbone dont le noyau aromatique est éventuellement substitué par un ou plusieurs atomes d'halogène ou un ou plusieurs groupes hydroxyle, alcoxyle de 1 ou 2 atomes de carbone, trifluorométhyle ou nitro.

Dans un mode de réalisation préférentiel de l'invention, la formule (I) définie ci-dessus est représentée par la formule (VIII) suivante : 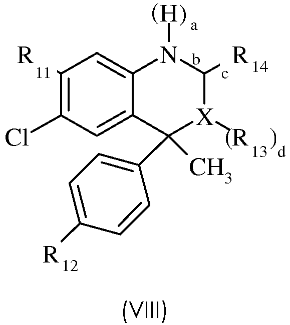 dans laquelle :
- a représente 0 ou 1 ;
- b représente une simple liaison ou une double liaison ;
- c représente une simple liaison ou une double liaison ;
- d représente 0 ou 1 ;
- X représente un atome d'oxygène ou d'azote, sous réserve que lorsque X représente un atome d'oxygène alors d vaut 0 et que lorsque X représente un atome d'azote alors d vaut 1 ;
- R₁₁ et R₁₂ identiques ou différents représentent -H ou -OH ;
- R₁₃ représente -H ou un groupe -CH₂-CH₃ ;
- R₁₄ représente un atome d'oxygène ou un groupe -NH₂ ou -NH-CH₂-CH₃, sous réserve que lorsque R₁₄ représente un atome d'oxygène alors a vaut 1, b représente une simple liaison et c représente une double liaison et que lorsque R₁₄ représente un groupe -NH₂ ou -NH-CH₂-CH₃ alors a vaut 0, b représente une double liaison et c représente une simple liaison.

Comme on l'entend ici, la formule (VIII) définie ci-dessus englobe notamment les formules des composés de formule (VIII) optiquement actifs, tels que les énantiomères représentés par les formules suivantes : 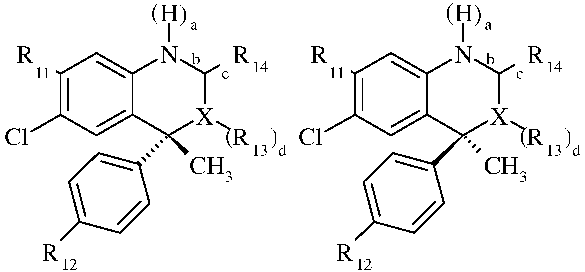 ou leurs mélanges, en particulier leur mélange racémique.

Dans un autre mode de réalisation préférentiel de l'invention, la formule (I) définie ci-dessus est représentée par la formule (II) suivante : 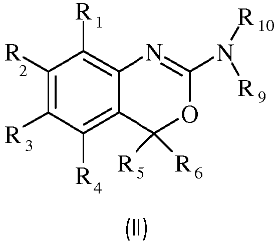 dans laquelle R₁, R₂, R₃, R₄, R₅, R₆, R₉ et R₁₀ sont tels que définis ci-dessus.

Comme on l'entend ici, la formule (II) définie ci-dessus englobe notamment les formules des composés de formule (II) optiquement actifs, tels que les énantiomères représentés par les formules suivantes (lorsque R₅ et R₆ sont différents) : 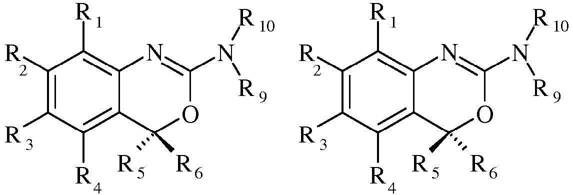 ou leurs mélanges, en particulier leur mélange racémique.

Dans un autre mode de réalisation préférentiel de l'invention, la formule (I) définie ci-dessus est représentée par la formule (IIIa), (IIIb) ou (IV) suivante :

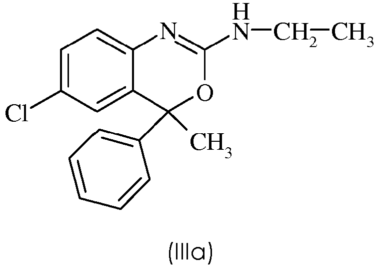

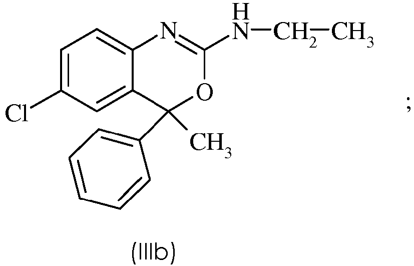

HCl

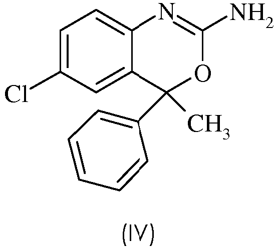

Le composé de formule (IIIa) est l'étifoxine base ou 6-chloro-2-éthylamino-4-méthyl-4-phényl-4H-[3,1]benzoxazine. Le composé de formule (IIIb) est l'étifoxine, ou chlorhydrate de 6-chloro-2-éthylamino-4-méthyl-4-phényl-4H-[3,1]benzoxazine.

Le composé de formule (IV), la déséthyl-étifoxine ou 2-amino-6-chloro-4-méthyl-4-phényl-4H-[3,1]benzoxazine, est un métabolite de l'étifoxine.

Comme on l'entend ici, la formule (IIIa) ou (IIIb) définie ci-dessus englobe notamment les formules des composés de formule (IIIa) ou (IIIb) optiquement actifs, tels que les énantiomères représentés par les formules suivantes : 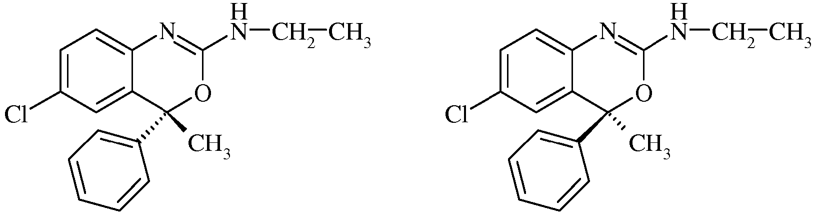 ou leurs mélanges, en particulier leur mélange racémique, notamment sous forme chlorhydrate, ainsi que les formules des composés de formule (IV) optiquement actifs, tels que les énantiomères représentés par les formules suivantes : 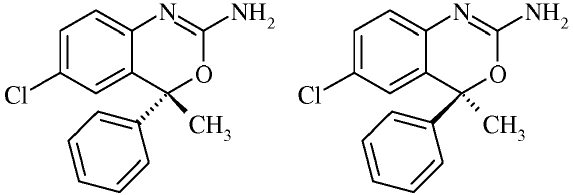 ou leurs mélanges, en particulier leur mélange racémique.

Dans un autre mode de réalisation préférentiel de l'invention, la formule (I) est représentée par la formule (V) suivante : 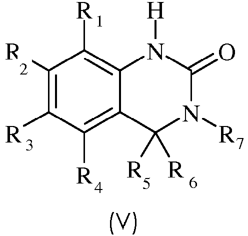 dans laquelle R₁, R₂, R₃, R4, R₅, R₆, et R₇ sont tels que définis ci-dessus.

Comme on l'entend ici, la formule (V) définie ci-dessus englobe notamment les formules des composés de formule (V) optiquement actifs, tels que les énantiomères représentés par les formules suivantes (lorsque R₅ et R₆ sont différents) : 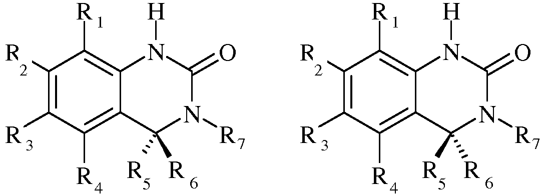 ou leurs mélanges, en particulier leur mélange racémique.

Dans un autre mode de réalisation préférentiel de l'invention, la formule (I) est représentée par la formule (VI) ou (VII) suivante :

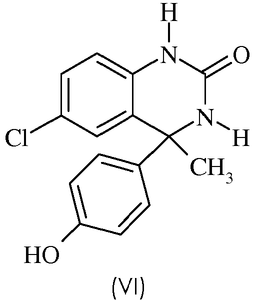

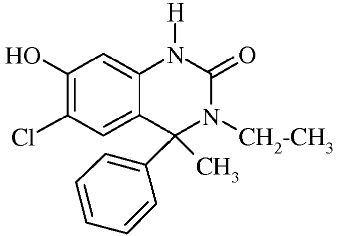

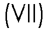

Les composés de formule (VI) (6-chloro-4-(4-hydroxy-phényl)-4-méthyl-3,4-dihydro-1H-quinazolin-2-one) et (VII) (6-chloro-3-éthyl-7-hydroxy-4-méthyl-4-phényl-3,4-dihydro-1H-quinazolin-2-one) sont des métabolites de l'étifoxine.

Comme on l'entend ici, la formule (VI) définie ci-dessus englobe notamment les formules des composés de formule (VI) optiquement actifs, tels que les énantiomères représentés par les formules suivantes : 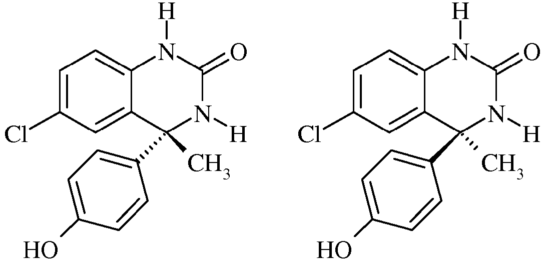 ou leurs mélanges, en particulier leur mélange racémique, ainsi que les formules des composés de formule (VII) optiquement actifs, tels que les énantiomères suivants : 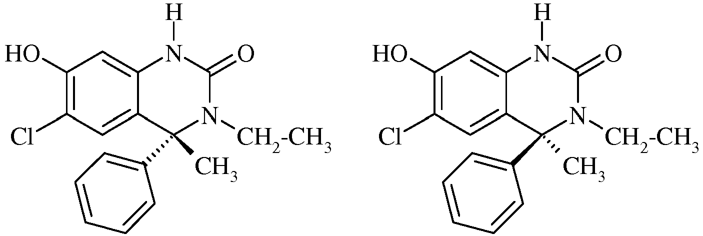 ou leurs mélanges, en particulier leur mélange racémique.

### Composé additionnel

L'autre composé utile pour la prévention ou le traitement de la maladie d'Alzheimer selon l'invention peut être de tout type, cependant on préfère qu'il soit sélectionné dans le groupe constitué du donépézil, de la galantamine, de la mémantine, et de la rivastigmine.

### Administration

De préférence, le composé de formule (I) telle que définie ci-dessus, ou son sel pharmaceutiquement acceptable, est administré ou administrable à une dose unitaire, ou est conditionné en une dose unitaire, d'environ 50 mg à environ 1500 mg, notamment d'environ 150 à environ 200 mg. De préférence également, le composé de formule (I) telle que définie ci-dessus, ou son sel pharmaceutiquement acceptable est administré ou administrable avec un régime de dosage de 50 mg/j à environ 1500 mg/j, notamment d'environ 150 mg/j à environ 200 mg/j.

De préférence, le composé de formule (I) telle que définie ci-dessus, ou son sel pharmaceutiquement est administré ou administrable sous une forme convenant pour une administration par voie orale. Plus préférablement, le composé de formule (I) telle que définie ci-dessus, ou son sel pharmaceutiquement acceptable, est administré ou administrable sous la forme d'une poudre, de cachets, de gélules ou de sachets.

Comme on l'entend ici l'expression « en combinaison » ou « produit de combinaison » signifie que le composé de formule (I) telle que définie ci-dessus, ou un sel pharmaceutiquement acceptable de celui-ci, et l'autre composé utile pour la prévention ou le traitement de la maladie d'Alzheimer tel que défini ci-dessus peuvent être associés au sein d'une même composition pharmaceutique ou d'un même médicament, et donc être administrés ensemble, ou bien être administrés de manière séparée, c'est-à-dire selon des voies d'administration distinctes et/ou des régimes d'administration distincts, sous réserve que lorsqu'ils sont administrés de manière séparée les périodes du composé de formule (I) telle que définie ci-dessus et de l'autre composé utile pour la prévention ou le traitement de la maladie d'Alzheimer tel que défini ci-dessus se recouvrent en totalité ou en partie.

Ainsi, lorsque les composés sont administrés de manière séparée, le composé de formule (I) telle que définie ci-dessus, ou son sel pharmaceutiquement acceptable, sera de préférence administré dans les 24 heures, plus préférablement dans les 2 heures, et encore plus préférablement dans l'heure, suivant l'administration de l'autre composé utile pour la prévention ou le traitement de la maladie d'Alzheimer tel que défini ci-dessus, et son administration sera éventuellement poursuivie les jours suivants. Réciproquement, l'autre composé utile pour la prévention ou le traitement de la maladie d'Alzheimer tel que défini ci-dessus sera de préférence administré dans les 24 heures, plus préférablement dans les 2 heures, et encore plus préférablement dans l'heure, suivant l'administration du composé de formule (I) telle que définie ci-dessus, ou de son sel pharmaceutiquement acceptable, et son administration sera éventuellement poursuivie les jours suivants. Dans un autre mode de réalisation préférée de l'invention, lorsque le composé de formule (I) telle que définie ci-dessus et l'autre composé utile pour la prévention ou le traitement de la maladie d'Alzheimer tel que défini ci-dessus sont administrés de manière séparée, ils sont administrés essentiellement simultanément.

L'invention sera davantage explicitée à l'aide des Exemples et des Figures non limitatifs qui suivent.

### Description des figures

Figure 1
   Effet de l'étifoxine sur l'altération de la mémoire spatiale induite par l'administration du peptide Aβ₂₅₋₃₅ chez les souris. Les doses sont exprimées en mg/kg par jour. 12 souris/ groupe ont été utilisées. Le symbole (***) représente p < 0.001 par rapport aux groupes traités avec Sc.Aβ + véhicule. Le symbole (###) représente p < 0.001 par rapport aux groupes traités avec le peptide Aβ₂₅₋₃₅.
Figure 2
   Effet de l'étifoxine sur l'augmentation de la peroxydation lipidique induite par le peptide Aβ₂₅₋₃₅. dans des hippocampes de souris. Les doses sont exprimées en mg/kg par jour. 6 animaux/groupe ont été utilisés. Le symbole (***) représente p < 0.001 par rapport aux groupes traités avec Sc.Aβ + véhicule. Les symboles (##) et (###) représentent respectivement p < 0.01 et p < 0.001 par rapport aux groupes traités avec Aβ25-35.
Figure 3
   Effet de l'étifoxine sur les neurones corticaux après intoxication par le peptide β-amyloïde 42. Chaque valeur représente la moyenne ± esm (100% = pas de β-amyloïde). β-amyloïde vs étifoxine, les symboles (*), (**) et (***) représentent respectivement p < 0.05, p < 0.01, p <0.001. L'estradiol (100nM) est le composé de référence.
Figure 4
   Effet de l'étifoxine à 7 concentrations différentes, sur le stress oxydatif induit par le peptide β-amyloïde 42, dans les neurones corticaux. Chaque valeur représente la moyenne ± esm (100% = pas de B-amyloïde). β-amyloïde vs étifoxine et BDNF, les symboles (*), (**) et (***) représentent respectivement p < 0.05, p < 0.01 et p <0.00. β-amyloïde par rapport au témoin, le symbole (#) représente p<0.01. Le BDNF (50 ng/mL) est le composé de référence.
Figure 5
   Effet de l'étifoxine à 7 concentrations différentes, sur l'hyperphosphorylation de la protéine tau, induite par le peptide β-amyloïde 42, dans les neurones corticaux. Chaque valeur représente la moyenne ± esm (100% = pas de β-amyloïde). β-amyloïde vs étifoxine et BDNF, les symboles (*), (**), et (***) représentent respectivement p < 0.05, p < 0.01 et <0.001. β-amyloïde par rapport au témoin, le symbole (#) représente p<0.01. Le BDNF (50ng/mL) est le composé de référence.

### Exemple 1: Evaluation in vivo de l'effet de l'étifoxine dans le modèle murin de maladie d'Alzheimer par administration du peptide amyloïde β₂₅₋₃₅.

Cet exemple a pour objectif l'évaluation *in vivo* de l'effet de l'étifoxine dans un modèle murin de maladie d'Alzheimer par administration du peptide amyloïde _{β25-35} (Maurice et al. (1996) Brain Res. 706:181-193 ; Maurice et al. (1998) Neuroscience 83:413-428 ; Meunier et al. (2006) J. Pharmacol. Exp. Ther. 317:1307-1319 ; Meunier et al. (2013) Genome Research 23:34-45 ; Villard et al. (2009) Neurophsychopharmacologie 34:1552-1566 ; Villard et al. (2011) J. Psychopharmacol. 25:1101-1117).

### A. Matériels et méthodes

### 1. Animaux

Des souris males âgées de 5 semaines et de poids compris entre 30 et 35 grammes (JANVIER, Saint Berthevin, France) sont utilisées. Les souris sont réparties en groupes et ont accès à la nourriture et à l'eau ad *libitum,* excepté pendant les expériences comportementales. Les animaux sont maintenus à température et humidité contrôlées avec des cycles lumière/obscurité de 12h/12h (la lumière est éteinte à 19 heures). Toutes les procédures animales sont réalisées en respectant strictement la directive de l'Union Européenne du 22 septembre 2010 (2010/63/UE).

### 2. Protocole

Soixante-douze souris males Swiss (30-35 grammes) sont utilisées. Six groupes sont constitués et soumis à différents traitements :

**Tableau 1. Groupes de traitement.**

| | **n** |
|---|---|
| 1. Sc.Aβ + véhicule, IP | 12 |
| 2. Aβ₂₅₋₃₅ + véhicule, IP | 12 |
| 3. Aβ₂₅₋₃₅ + composé de référence (Denepezil, 1 mg/kg), IP | 12 |
| 4. Aβ₂₅₋₃₅ + Etifoxine, 12,5 mg/kg, IP | 12 |
| 5. Aβ₂₅₋₃₅ + Etifoxine, 25 mg/kg, IP | 12 |
| 6. Aβ₂₅₋₃₅ + Etifoxine, 50 mg/kg, IP | 12 |
| Nombre total de souris | 72 |

Le 1^{er} jour : une version « mélangée » (scrambled) du peptide amyloïde _{β25-35} (Sc.Aβ, témoin négatif) ou le peptide amyloïde _{β25-35} (Aβ₂₅₋₃₅) est injecté par voie intra-cérébroventriculaire (ICV) (le peptide Aβ₂₅₋₃₅ provoque l'intoxication cellulaire). Entre le 1^{er} jour et le 10^{ème} jour, les traitements sont administrés une fois par jour par voie intrapéritonéale (IP).

Du 8^{ème} au 10^{ème} jour, deux tests comportementaux sont effectués :
- Procédure d'alternance spontanée dans le labyrinthe en Y (évaluation de la mémoire spatiale de travail) le 8^{ème} jour, soit 7 jours après l'injection de peptide ;
- Réponse d'évitement passif (évaluation de la mémoire contextuelle à long terme) avec l'entrainement le 9^{ème} jour et la séance de rétention le 10^{ème} jour.

A la fin de la session de rétention d'évitement passif, le 10^{ème} jour, les animaux sont sacrifiés par décapitation. L'hippocampe et le cortex frontal sont disséqués et congelés dans l'azote liquide. L'hippocampe est utilisé pour mesurer le taux de peroxydation lipidique à l'aide d'une méthode colorimétrique et pour évaluer les taux de 4 marqueurs biochimiques par des tests ELISA. Le cortex est conservé à -80°C en attente d'analyses supplémentaires. Les plasmas sont également conservés en attente d'analyses supplémentaires. Les échantillons biologiques sont jetés après 3 mois.

### 3. Produits

### 3.1. Composés de référence :

L'étifoxine est fournie par Biocodex sous forme de poudre et est conditionnée dans 8 bouteilles en verre. L'étifoxine est solubilisée dans NaCl à 0,9% et du Tween 80 et est administrée par voie intrapéritonéale aux doses indiquées (100 µL/20 g de poids corporel).

Le donépézil est solubilisé dans du NaCl à 0,9%
- Dénomination : donépézil
- Nom IUPHAR : 2-[(1-benzyl-4-piperidyl)methyl]-5,6-dimethoxy-2,3-dihydroinden-1-one, hydrochloride, hydrate (1:1:1)
- CAS : 120014-06-4
- Fournisseur : Sigma-Aldrich (France)
- Référence : D6821

### Peptides β-amyloïde :

Aβ25-35 :
   - Dénomination : *amyloid-β protein* (25-35), *human, mouse, rat*
   - CAS : 131602-53-4
   - Fournisseur : Polypeptides (France)
   - Référence : SC489
Sc.Aβ :
   - Dénomination : *Scrambled Amyloid-β Protein* (25-35), *human, mouse, rat*
   - CAS : NA
   - Fournisseur : Polypeptides (France)
   - Référence : SC942

### 3.2. Administration des peptides amyloïdes

Chaque souris est anesthésiée avec 2,5% d"isoflurane. Le peptide Aβ₂₅₋₃₅ (9 nmol/souris) ou le peptide Sc.Aβ (9 nmol/souris) est injecté par voir ICV, dans un volume final de 3 µL/souris. Ces injections permettent d'établir un modèle murin de la maladie d'Alzheimer (Maurice et al. (1996) Brain Res. 706:181-193 ; Maurice et al. (1998) Neuroscience 83:413-428 ; Meunier et al. (2006) J. Pharmacol. Exp. Ther. 317:1307-1319 ; Meunier et al. (2013) Genome Research 23:34-45 ; Villard et al. (2009) Neurophsychopharmacologie 34:1552-1566 ; Villard et al. (2011) J. Psychopharmacol. 25:1101-1117).

### 4. Déroulement des tests

### 4.1. Analyse comportementale et biochimique

### 4.1.1. Test d'alternance spontanée

Le 8^{ème} jour, les animaux sont soumis à des tests d'alternance spontanée dans le labyrinthe en Y pour indexer le travail de la mémoire spatiale. Le labyrinthe en Y est constitué de chlorure de polyvinyle (PVC) gris et compte 3 bras ayant une même origine. Chaque bras mesure 40 cm de long, 13 cm de haut, 3 cm de large à la base, 10 cm de large en haut. Les angles formés entre chaque bras sont égaux. Chaque souris est placée à l'extrémité d'un bras et peut se déplacer librement à travers le labyrinthe pendant une session de 8 minutes (min.). Le nombre d'entrées dans chaque bras ainsi que les éventuels retours dans le même bras, sont comptés visuellement. Une alternance est comptabilisée lorsqu'une entrée est effectuée dans les 3 bras lors d'essais successifs. Le nombre d'alternances maximales possibles est donc le nombre total d'entrées dans les branches moins 2 et le pourcentage d'alternance est calculé comme suit : (alternances comptabilisées / alternances maximales) x 100. Les paramètres incluent le pourcentage d'alternance (indice de la mémoire) et le nombre total d'entrées dans les branches (indice d'exploration) (Maurice et al. (1996) Brain Res. 706:181-193 ; Maurice et al. (1998) Neuroscience 83:413-428 ; Meunier et al. (2006) J. Pharmacol. Exp. Ther. 317:1307-1319 ; Meunier et al. (2013) Genome Research 23:34-45 ; Villard et al. (2009) Neurophsychopharmacologie 34:1552-1566 ; Villard et al. (2011) J. Psychopharmacol. 25:1101-1117). Les animaux présentant un comportement extrême (pourcentage d'alternance < 20% ou > 90% ou un nombre d'entrées dans le bras < 10) sont écartés du calcul.

### 4.1.2. Test d'évitement passif

L'appareil du test est une boite à deux compartiments (15 x 20 x 15 cm de haut). Un compartiment avec des parois en polychlorure de vinyle (PVC) blanc est éclairé et l'autre avec des parois en polyvinyle de chlorure noir et un sol grillagé est sombre. Une trappe sépare chaque compartiment. Une lampe de 60 W est placée à 40 cm au-dessus du dispositif et éclaire le compartiment blanc pendant l'expérience. Au niveau de la grille, des chocs électriques aléatoires de 0,3 mA sont délivrés aux niveaux des pattes des souris pendant 3 secondes grâce à un générateur électrique aléatoire (Lafayette Instruments, Lafayette, États-Unis). Pendant les sessions d'entrainement la trappe est initialement fermée. Au début de l'entrainement chaque souris est placée dans le compartiment blanc. La trappe est levée après 5 secondes. Lorsque la souris entre dans le compartiment sombre et appuie toutes ses pattes sur la grille, la trappe est fermée et le choc électrique aléatoire est délivré aux pattes pendant 3 secondes. La latence de franchissement, à savoir, le temps de latence avant l'entrée dans le compartiment sombre et le nombre de vocalisations sont enregistrés. Le test de rétention est effectué 24 heures après l'entrainement. Chaque souris est à nouveau placée dans le compartiment blanc. Après 5 secondes, la trappe est levée. Le temps de latence et le temps de fuite (correspondant à la durée avant la sortie du compartiment sombre) sont enregistrés pendant 300 secondes (Meunier et al. (2006) J. Pharmacol. Exp. Ther. 317:1307-1319 ; Villard et al. (2009) Neurophsychopharmacologie 34:1552-1566 ; Villard et al. (2011) J. Psychopharmacol. 25:1101-1117).

Les animaux pour lesquels les temps de latences lors des sessions d'entrainements et de rétentions sont inférieurs à 10 secondes sont écartés des calculs. Dans cette étude, aucun animal n'a été écarté.

### 4.2. Mesure de la peroxydation lipidique

Les souris de chaque groupe sont sacrifiées par décapitation et les deux hippocampes sont prélevés, pesés et conservés dans de l'azote liquide jusqu'à l'analyse. Un hippocampe par souris est décongelé et homogénéisé dans du méthanol froid (1/10 w/v), centrifugé à 1000 grammes pendant 5 minutes et le surnageant est placé dans un tube Eppendorf. Le volume de réaction de chaque homogénat est ajouté à FeSO4 (1 mM), H2SO4 (0,25 M), orange de xynlénol (1 mM) et incubé pendant 30 minutes à température ambiante. Après avoir lu l'absorbance à 580 nm (A₅₈₀1), 10 µl d'hyperoxyde de cumène (CHP) (1 mM) sont ajoutés à l'échantillon et il est incubé pendant 30 minutes à température ambiante pour déterminer le degré d'oxydation maximal. L'absorbance est mesurée à 580 nm (A₅₈₀2). Le niveau de peroxydation des lipides est déterminé en équivalent de CHP selon la formule : CHPE = A₅₈₀1 /A₅₈₀2 x [CHP (nmol)] et exprimé en équivalents de CHP par mg de tissu et en pourcentage par rapport au témoin (Sc.Aβ + véhicule).

### 4.3. Tests ELISA

### Les taux de Bax, Bcl2, GFAP et Caspase-3 sont analysés à l'aide de tests ELISA :

### Dysfonctionnement mitochondrial / apoptose

| | | |
|---|---|---|
| Caspase-3 : | Fournisseur : USCNK | Référence : SEA626Mu |
| Bax : | Fournisseur : USCNK | Référence: SEB343Mu |
| Bcl2 : | Fournisseur : USCNK | Référence : SEA778Mu |

### Processus inflammatoires

| | | |
|---|---|---|
| GFAP : | Fournisseur : USCNK | Référence : SEA425Mu |

Pour tous les dosages, les cortex sont homogénéisés, après décongélation, dans un tampon Tris (Tris 50 mM, 150 mM NaCl, pH 7,5) et traités par ultrasons pendant 20 secondes. Après centrifugation (16,100 grammes pendant 15 minutes à 4 ° C), les surnageants sont utilisés pour les tests ELISA selon les instructions du fabricant. Pour chaque dosage, l'absorbance est lue à 450 nm et la concentration est calculée en utilisant la courbe standard. Les résultats sont exprimés en pg ou ng de marqueur par mg de tissu et en pourcentage de solution Sc.Aβ + véhicule.
6 échantillons par groupe (n = 36 kits ELISA) sont dosés en double.

### B. Résultats

### 1. Test d'alternance spontanée

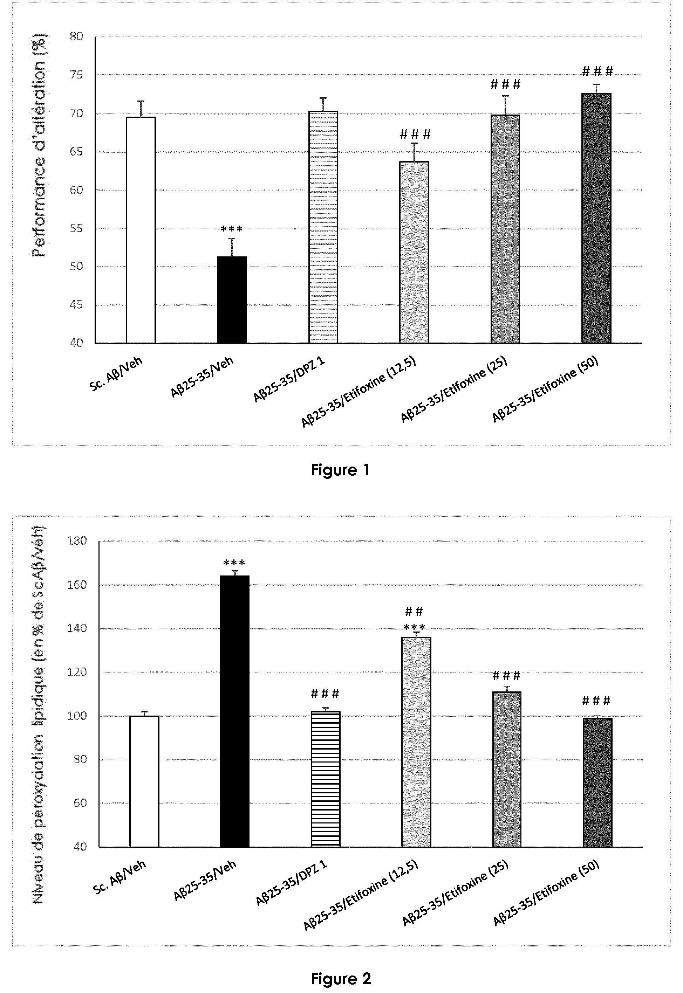

### Les résultats sont présentés dans la Figure 1.

L'injection du peptide Aβ₂₅₋₃₅ altère de manière significative la mémoire spatiale par comparaison avec les souris ayant reçu le peptide témoin négatif (Sc.Aβ + Véhicule). Les résultats démontrent que l'administration d'étifoxine atténue de façon significative les déficits d'apprentissage à toutes les doses testées.

### 2. Test d'évitement passif

Le **tableau 2A** présente les temps de latence et le **tableau 2B** présente les temps de fuite lors de la session de rétention pendant le test d'évitement passif. L'injection du peptide Aβ₂₅₋₃₅ altère de manière significative la mémoire contextuelle à long terme (temps de latence diminué, temps de fuite augmenté), par comparaison aux souris traitées avec le peptide témoin (Sc.Aβ + Véhicule).

Les résultats montrent que l'administration d'étifoxine améliore les deux critères testés. Des profils similaires ont été observés pour le temps de latence et le temps de fuite.

**Tableau 2A : Mesure du temps de latence lors la session de rétention.**

| Sc. Aβ/Véh (nmol) | Aβ₂₅₋₃₅/Véh (nmol) | Donépézil (mg/kg) | Etifoxine (mg/kg) | Moyenne (%) | Valeur P |
|---|---|---|---|---|---|
| 9 | 0 | 0 | 0 | 252,8 ± 10,4 | |
| 0 | 9 | 0 | 0 | 110,9 ± 10,1 | P<0,001 |
| 0 | 9 | 1 | 0 | 246,5 ± 13,4 | P<0,001 |
| 0 | 9 | 0 | 12,5 | 208,1 ± 17,4 | |
| 0 | 9 | 0 | 25 | 234,7 ± 14,1 | P<0,01 |
| 0 | 9 | 0 | 50 | 251,5 ± 15,6 | P<0,001 |

**Tableau 2B : Mesure du temps de fuite lors de la session de rétention.**

| Sc. Aβ/Véh (nmol) | Aβ₂₅₋₃₅/Véh (nmol) | Donépézil (mg/kg) | Etifoxine (mg/kg) | Moyenne (%) | Valeur P |
|---|---|---|---|---|---|
| 9 | 0 | 0 | 0 | 21,8 ± 2,6 | |
| 0 | 9 | 0 | 0 | 66,8 ± 6,1 | P<0,001 |
| 0 | 9 | 1 | 0 | 19,9 ± 2,5 | P<0,001 |
| 0 | 9 | 0 | 25 | 22,4 ± 2,4 | P<0,001 |
| 0 | 9 | 0 | 50 | 21,6 ± 3,0 | P<0,001 |

### 3. Mesure de la peroxydation lipidique

Les résultats sont présentés dans la **Figure 2****.** L'injection du peptide Aβ₂₅₋₃₅ augmente de manière significative le niveau de peroxydation lipidique dans l'hippocampe des souris par comparaison aux souris traitées avec le peptide témoin négatif (Sc.Aβ + Véhicule). L'administration d'étifoxine permet de diminuer de manière dose-sensible le stress oxydatif induit par l'administration du peptide Aβ₂₅₋₃₅.

### 4. Tests ELISA

### Les résultats sont résumés dans le Tableau 3

**Tableau 3 : Effet du traitement par l'étifoxine sur les taux de Bax, Bcl2, GFAP et Caspase-3 dans les hippocampes de souris. (¹ V = véhicule).**

| Traitement | Taux de protéine Bax (% de Sc.Aβ/V) | Taux de protéine Bcl2 (% de Sc.Aβ/V) | Ratio Bax/Bcl2 (% de Sc.Aβ/V) | Expression CASP-3 (% de Sc.Aβ/V) | Expression GFAP (% de Sc.Aβ/V) |
|---|---|---|---|---|---|
| Sc.Aβ/V¹ | 100 ± 5,6 | 100 ± 4,7 | 100 ± 8,2 | 100 ± 7,3 | 100 ± 2,3 |
| Aβ₂₅₋₃₅/V | 189,7 ± 6 | 100,3 ± 3,6 | 187,6 ± 8,6 | 168,1 ± 6,1 | 221,1 ± 8,9 |
| Aβ₂₅₋₃₅/DPZ 1 | 95,7 ± 4,3 | 100,3 ± 3 | 91,5 ± 3,5 | 98,7 ± 4,3 | 110,3 ± 6,8 |
| Aβ_{25- 35}/Etifoxine 12.5 mg/kg | 180,1 ± 7,4 | 97,2 ± 2,1 | 182,7 ± 7 | 136,8 ± 7,2 | 160,4 ± 10,2 |
| Aβ_{25- 35}/Etifoxine 25 mg/kg | 132,6± 7,5 | 99,4 ± 2,3 | 135,5 ± 10,2 | 127,3 ± 10,8 | 131,7 ± 4,3 |
| Aβ_{25- 35}/Etifoxine 50 mg/kg | 99,9 ± 4,2 | 102,3 ± 3,2 | 97,3 ± 7 | 109,9 ± 5,6 | 129,8 ± 2,7 |

L'injection du peptide Aβ₂₅₋₃₅ entraine l'augmentation du taux de la protéine pro-apoptotique Bax, du rapport Bax/Bcl2, du taux de caspase-3 et de l'expression de GFAP dans l'hippocampe de souris, par rapport à des souris ayant reçu le peptide témoin négatif (Sc.Aβ + Véhicule).

L'injection de du peptide Aβ₂₅₋₃₅ n'a aucun effet sur le taux de la protéine anti-apoptotique Bcl2.

L'administration de l'étifoxine :
- bloque de manière dose-dépendante l'apoptose en réduisant le rapport Bax/Bcl2 et le taux de caspase-3 ;
- n'exerce aucun effet sur le taux de Bcl2 ;
- réduit l'augmentation du taux de protéine GFAP induite par l'administration de Aβ₂₅₋₃₅.

### Exemple 2 : Evaluation in vitro de l'effet de l'étifoxine dans le modèle de maladie d'Alzheimer d'intoxication de neurones corticaux par le peptide amyloïde β₁₋₄₂.

Cet exemple a pour objectif l'évaluation *in vitro* de l'effet de l'étifoxine dans un modèle de maladie d'Alzheimer d'intoxication de neurones corticaux par le peptide amyloïde β₁₋₄₂ (Callizot et al. (2013) J. Neurosci. Res. 9 :706-16).

### A. Matériels et méthodes

### 1. Neurones corticaux

Des neurones corticaux de rats sont mis en culture comme dans la méthode décrite par Singer (1999) J. Neurosci. 19:2455-2463. Des rats femelles enceintes de 15 jours sont tuées par dislocation des cervicales (Rats Wistar, Janvier) et le fœtus est retiré de l'utérus. Les cortex sont prélevés et placés dans un milieu glacé de Leibovitz (L15, Panbiotech, Réf : P04-27055, lot : 9310614) contenant 2% de pénicilline (10 000 U/ml) et de streptomycine (10 mg/ml) (PS, Panbiotec, Réf : P06-07100, lot : 48101114) et 1% de sérum-albumine bovine (BSA, Panbiotech, Réf : P06-1391100, lot : H140603). Les cortex sont dissociés par trypsination (0,05% Panbiotech, Réf : P10-023100, lot : 5890314) pendant 20 minutes à 37°C. La réaction est stoppée par addition d'un milieu Eagle modifié de Dulbecco (DMEM, Panbiotech, réf P04-03600, lot : 9670913) contenant de la DNase I grade Il (0,1 mg/ml Panbiotech, Réf : P60-37780100, lot : H140508) et 10 % de sérum de veau fœtal (FCS, Invitrogen, Réf : 10270 à 098, lot : 41Q4120K). Les cellules sont dissociées mécaniquement par 3 passages à travers une pipette de 10 ml. Les cellules sont ensuite centrifugées à 515 g pendant 10 minutes à 4°C. Le surnageant est éliminé et les cellules sont remises en suspension dans un milieu de culture Neurobasal (Nb, Invitrogen, Réf : 21103, lot : 1608692) supplémenté avec 2% de B27 (Invitrogen, Réf : 17504, lot : 1589889), 2 mM de L-glutamine (Panbiotech, Réf : P04-80100, lot : 6620314), 2% de la solution PS et 10 ng/ml de facteur neurotrophique dérivé du cerveau (BDNF, PanBiotech, Réf : CB-1115002, lot : 4810114). Les cellules sont ensemencées à une densité de 30 000 cellules/puits dans des plaques de 96 puits pré-enduites de poly-L-lysine (10 ng/ml, Greiner Réf : 655930, lot : E14021HD) et cultivées à 37 °C dans une atmosphère humidifiée avec 95% d'air et 5% de CO₂ pour l'évaluation de la survie des neurones (analyse de MAP2 par immuno-coloration) (6 puits/condition, 1 culture).

### 2. Préparation du peptide amyloïde B₁₋₄₂ humain

La préparation du peptide amyloïde β₁₋₄₂ (Aβ₁₋₄₂) est réalisée en suivant la procédure par Callizot et al. (2013) Neurosci. Res. 91:706-16. Brièvement, le peptide Aβ₁₋₄₂ humains est reconstitué dans le milieu de culture à 40 µM (solution mère) et est lentement agité dans l'obscurité pendant 3 jours à 37 °C. Le milieu témoin est préparé dans les mêmes conditions.

### 2.1. Préparation du peptide Aβ₁₋₄₂ humain pour l'évaluation de l'effet neuroprotecteur de l'étifoxine

Après 3 jours, 10 µM de la solution de peptide amyloïde sont incubés pendant 24 heures sur les neurones corticaux puis dilués dans un milieu témoin. De l'estradiol (100nM) et l'étifoxine sont dissouts dans le milieu de culture et pré-incubés pendant 1 heure avant l'ajout du peptide Aβ₁₋₄₂ agrégé.

### 2.2. Préparation du peptide Aβ₁₋₄₂ humain pour l'évaluation de l'effet de l'étifoxine sur le stress oxydatif

Après 3 jours, 1,25 µM de la solution de peptide amyloïde sont incubés pendant 4 heures sur les neurones corticaux puis dilués dans le milieu témoin. Le BDNF (50 ng/mL) et l'étifoxine sont dissouts dans le milieu de culture et pré-incubés pendant 1 heure avant l'ajout du peptide Aβ₁₋₄₂ agrégé.

### 2.3. Préparation du peptide Aβ₁₋₄₂ humains pour l'évaluation de l'effet de l'étifoxine sur l'hyperphosphorylation de la protéine Tau

Après 3 jours, 2,5 µM de la solution de peptide amyloïde sont incubés pendant 16 heures sur les neurones corticaux puis dilués dans le milieu témoin. Le BDNF (50 ng/mL) et l'étifoxine sont dissouts dans le milieu de culture et pré-incubés pendant 1 heure avant l'application du peptide Aβ₁₋₄₂ agrégé.

### 3. Traitement

### 3.1. Traitement pour l'évaluation de l'effet neuroprotecteur de l'étifoxine

Après 11 jours de culture les cellules sont pré-incubées avec de l'estradiol (100 nM) et de l'étifoxine pendant 1 heure avant l'ajout du peptide Aβ₁₋₄₂ agrégé. 1 heure après le traitement, les cellules sont intoxiquées avec 10 µM de peptide Aβ₁₋₄₂ (Bachem, France) en présence des composés tests. De l'estradiol (100 nM) est utilisé comme composé de référence. Une culture par condition est réalisée.

### 3.2. Traitement pour l'évaluation de l'effet de l'étifoxine sur le stress oxydatif

Après 11 jours de culture, les cellules sont pré-incubées avec du BDNF (50 ng/mL) ou de l'étifoxine pendant 1 heure avant l'ajout du peptide Aβ₁₋₄₂ agrégé. 1 heure après le traitement, les cellules sont intoxiquées avec 1,25 µM de peptide Aβ₁₋₄₂ (Bachem, France) en présence des composés tests. Le BDNF (50 ng/mL) est utilisé comme composé de référence. Une culture par condition est réalisée.

### 3.3. Traitement pour l'évaluation de l'effet de l'étifoxine sur l'hyperphosphorylation de la protéine Tau

Après 11 jours de culture, les cellules sont pré-incubées avec du BDNF (50 ng/mL) ou de l'étifoxine pendant 1 heure avant l'application du peptide Aβ₁₋₄₂ agrégé. 1 heure après le traitement médical, les cellules sont intoxiquées avec 2,5 µM de peptide Aβ₁₋₄₂ (Bachem, France) en présence des composés tests. Le BDNF (50 ng/mL) est utilisé comme composé de référence. Une culture par condition est réalisée.

### 4. Evaluation de la neuroprotection

Les conditions expérimentales suivantes ont été testées :

**Tableau 4 : Conditions expérimentales pour l'évaluation de la neuroprotection.**

| Evaluation de l'effet neuroprotecteur de l'étifoxine | Evaluation de l'effet de l'étifoxine sur le stress oxydatif | Evaluation de l'effet de l'étifoxine sur l'hyperphosphorylation de la protéine Tau |
|---|---|---|
| Témoin (véhicule 0,1%) | Témoin (véhicule 0,1%) | Témoin (véhicule 0,1%) |
| Peptide Aβ₁₋₄₂ à 10 µM, 24 heures | Peptide Aβ₁₋₄₂ à 1,25 µM, 4 heures | Peptide Aβ₁₋₄₂ à 2,5 µM, 16 heures |
| Etifoxine (30µM, 10µM, 3µM, 1µM, 0,3µM, 0,1µM et 0,03µM) + Aβ₁₋₄₂ à 10 µM, 24 heures | Etifoxine (30µM, 10µM, 3µM, 1µM,) + Aβ₁₋₄₂ à 1,25 µM, 4 heures | Etifoxine (30µM, 10µM, 3µM, 1µM, 0,3µM, 0,1µM et 0,03µM) + Aβ₁₋₄₂ à 2,5 µM, 16 heures |
| Estradiol (100 nM) + Aβ₁₋₄₂ à 10 µM, 24 heures | BDNF (50 ng/mL) + Aβ₁₋₄₂ à 1,25 µM, 4 heures | BDNF (50 ng/mL) + Aβ₁₋₄₂ à 2,5 µM, 16 heures |

### 5. Déroulement des tests

### 5.1. Evaluation de la survie des cellules après administration du peptide Aβ₁₋₄₂

24 heures après l'intoxication, les neurones corticaux sont fixés par une solution froide d'Alcool (95%, Sigma, réf. 32221) et d'acide acétique (5%, Sigma, Réf : 33209, lot : 82420) pendant 5 minutes. Ensuite, les cellules sont perméabilisées et les sites non spécifiques sont bloqués avec une solution de PBS (PBS, PanBiotech, Réf : P04-36500) contenant 0,1% de saponine (Sigma Aldrich, Réf : S7900) et 1% de FCS pendant 15 minutes. Puis les cellules sont incubées avec un anticorps monoclonal de souris primaire dirigé contre la Protéine 2 Associée aux Microtubules (MAP-2, Sigma M4403) pendant 2 heures dans la même solution à la dilution de 1/400. Cet anticorps marque spécifiquement les corps cellulaires et les neurites des neurones. L'anticorps est révélé par un anticorps de chèvre anti-souris couplé à l'Alexa Fluor 488 (Molecular probe, Réf :1001) à 1/400 pendant 1 heure. Les noyaux des cellules sont marqués par un marqueur fluorescent (solution Hoechst, SIGMA Réf : B1155,). La survie neuronale totale est évaluée en comptant le nombre de corps cellulaires neuronaux positifs au marquage de MAP-2.

Pour chaque puits de culture, 10 images par puits sont prises à l'aide d'un système *Incell Analyzer TM* 2000 (GE Healthcare) avec un grossissement x 20. Toutes les images sont prises dans les mêmes conditions. Le nombre de neurones est automatiquement évalué à l'aide du logiciel Developer system analysis (GE Healthcare).

### 5.2. Effet de l'étifoxine sur le stress oxydatif après administration du peptide Aβ₁₋₄₂

4 heures après l'intoxication, les neurones corticaux sont fixés par une solution froide d'Alcool (95%, Sigma, Réf : 32221) et d'acide acétique (5%, Sigma, Réf : 33209) pendant 5 minutes. Ensuite, les cellules sont perméabilisées et les sites non spécifiques sont bloqués avec une solution de PBS (PBS, PanBiotech, Réf : P04-36500) contenant 0,1% de saponine (Sigma Aldrich, réf. S7900) et 1% de FCS pendant 15 minutes. Puis les cellules sont incubées avec un anticorps monoclonal de souris primaire dirigé contre la Protéine 2 Associée aux Microtubules (MAP-2, Sigma M4403) pendant une nuit dans la même solution à la dilution de 1/400. Cet anticorps marque spécifiquement les corps cellulaires et les neurites des neurones. Un co-marquage est effectuée à l'aide d'anticorps polyclonaux de lapin primaires anti-MetO à la dilution de 1/100 (Novus biological, Réf : NBP1-06707). Ces anticorps sont révélés avec un anticorps de chèvre (IgG) anti-souris couplé à l'Alexa Fluor 488 (Molécular probe, réf. A11001) et avec un anticorps (IgG) de chèvre anti-lapin couplé à l'Alexa Fluor 568 (Molecular Probe, réf. A11011) à 1/400 pendant 1 heure. Les noyaux des cellules sont marqués par un marqueur fluorescent (solution Hoechst, SIGMA Réf : B1155).

Pour chaque puits de culture, 10 images par puits sont prises à l'aide du système *Incell Analyzer TM* 2000 (GE Healthcare) avec un grossissement x 20. Toutes les images sont prises dans les mêmes conditions. L'analyse est faite à l'aide du logiciel Developer software (GE Healthcare) afin d'évaluer le chevauchement entre la coloration MAP-2 et le marquage MetO. Les résultats sont exprimés en nombre de neurones présentant un chevauchement par champ.

### 5.3. Effet de l'étifoxine sur l'hyperphosphorylation de la protéine Tau après administration du peptide Aβ₁₋₄₂

16 heures après l'intoxication, les neurones corticaux sont fixés par une solution froide d'Alcool (95%, Sigma, Réf : 32221) et d'acide acétique (5%, Sigma, Réf : 33209) pendant 5 minutes. Ensuite, les cellules sont perméabilisées et les sites non spécifiques sont bloqués avec une solution de PBS (PBS, PanBiotech, Réf : P04-36500) contenant 0,1% de saponine (Sigma Aldrich, Réf : S7900) et 1% de FCS pendant 15 minutes. Puis les cellules sont incubées avec un anticorps monoclonal de souris dirigé contre les filaments appariés en hélice (PHF) de la protéine Tau hyperphosphorylée (Fisher scientific, ref MN1060) pendant une nuit dans la même solution à la dilution de 1/400. Cet anticorps reconnait spécifiquement PHF-Tau phosphorylée au niveau de la Ser212 et de la Thr214. Un co-marquage est effectué en utilisant des anticorps polyclonaux primaires de poulet dirigés contre la Protéine Associée aux Microtubules 2 (MAP-2, Abcam, réf : ab5392) à une dilution de 1/400. Ces anticorps sont révélés par un anticorps de chèvre anti-souris couplé à l'Alexa Fluor 488 (Molecular probe, réf. A11001) et par un anticorps de chèvre anti-poulet (IgG) couplé à l'Alexa Fluor 568 (Molecular probe, réf. A11041) à une dilution de 1/400 pendant 1 heure. Les noyaux des cellules sont marqués par un marqueur fluorescent (solution Hoechst, SIGMA réf. B1155).

Pour chaque puits de culture, 10 images par puits sont prises à l'aide du système Incell *Analyzer* 2000 (GE Healthcare) avec un grossissement x 20. Toutes les images sont prises dans les mêmes conditions. La superficie de la protéine Tau phosphorylée est analysée et le rapport avec le nombre de corps cellulaires de neurones a été effectué.

### B. Résultats

### 1. Evaluation de la survie des cellules après administration du peptide Aβ₁₋₄₂

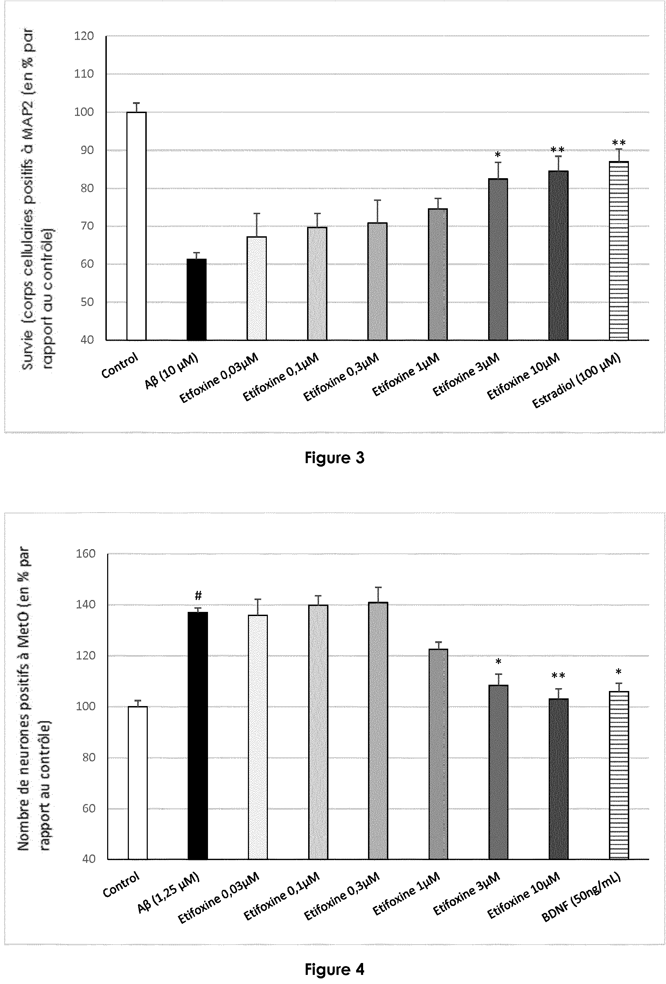

Les résultats sont présentés dans la **Figure 3****.** Le nombre de neurones corticaux diminue significativement en présence du peptide Aβ₁₋₄₂ (10 µM, 24 heures), à savoir de l'ordre de 60% par comparaison aux conditions témoin. Le niveau de marquage des neurones pour MAP-2 est restauré dans le cas d'une pré-incubation d'une heure avec de l'estradiol (100 nM).

L'étifoxine présente un effet neuroprotecteur sur les neurones corticaux intoxiqués par le peptide Aβ₁₋₄₂. En effet, l'étifoxine restaure de manière significative la survie des neurones à 10 µM (**, p <0,01) et 3 µM (*, p <0,05).

### 2. Effet de l'étifoxine sur le stress oxydatif après administration du peptide Aβ₁₋₄₂

Les résultats sont présentés sur la **Figure 4****.** Le peptide Aβ₁₋₄₂ (1,25 µM, 4 h) induit une augmentation importante du nombre de neurones ayant subi un stress oxydatif, c'est-à-dire positifs à MetO (136.98% du témoin, p <0,01). Le BDNF rétablit de manière significative le nombre de neurones positifs à MetO (105,95% du témoin, *, p <0,05). L'étifoxine à 3 µM et 10 µM, réduit significativement le nombre de neurones positifs à MetO (respectivement 108.40% du témoin, *, p <0,05 et 103,06% du témoin, **, p <0,01). Les résultats montrent donc que l'étifoxine réduit le stress oxydatif induit par le peptide Aβ₁₋₄₂.

### 3. Effet de l'étifoxine sur l'hyperphosphorylation de la protéine Tau après administration du peptide Aβ₁₋₄₂

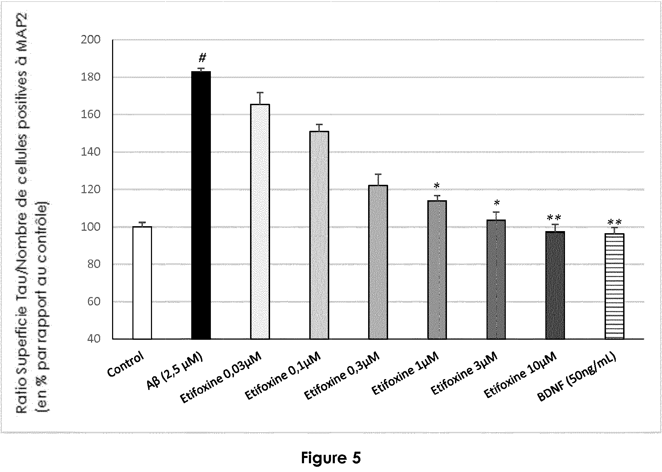

Les résultats sont présentés sur la **Figure 5****.** Le peptide Aβ₁₋₄₂ (2,5 µM, 16 h) induit une augmentation importante et significative de l'hyperphosphorylation de Tau (183% du témoin, #, p <0,01). Le BDNF (50 ng / ml) diminue l'hyperphosphorylation de Tau au niveau du témoin (96,3% du témoin, **, p <0,01). L'étifoxine à 1 µM (*, p <0,05), 3 µM (*, p <0,05) et 10 µM (**, p <0,01) rétablit de manière significative le niveau d'hyperphosphorylation de Tau à la valeur initiale (respectivement 113,91%, 103,57% et 97,38% du témoin).

L'étifoxine présente donc un effet protecteur des neurones corticaux vis-à-vis de l'hyperphosphorylation de Tau, induite par le peptide Aβ₁₋₄₂.

## Revendications

1. Composé de formule (I) suivante : 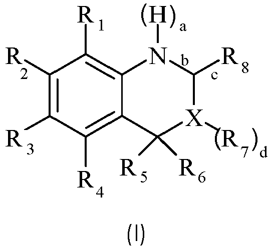 dans laquelle :
- a représente 0 ou 1 ;
- b représente une simple liaison ou une double liaison ;
- c représente une simple liaison ou une double liaison ;
- d représente 0 ou 1 ;
- X représente un atome d'oxygène ou d'azote, sous réserve que lorsque X représente un atome d'oxygène alors d vaut 0 et que lorsque X représente un atome d'azote alors d vaut 1;
- R₁, R₂, R₃, et R₄, identiques ou différents, représentent un atome d'hydrogène, un atome d'halogène, notamment choisi parmi F, Cl, Br, ou I, un groupe hydroxyle, ou un groupe alkoxyle de 1 ou 2 atomes de carbone ;
- R₅ et R₆, identiques ou différents, représentent un atome d'hydrogène, un groupe alkyle ou cycloalkyle de 1 à 6 atomes de carbone, ou un groupe aryle de 6 atomes de carbone dont le noyau aromatique est éventuellement substitué par un ou plusieurs atomes d'halogène ou un ou plusieurs groupes hydroxyle, alkoxyle de 1 ou 2 atomes de carbone, trifluorométhyle ou nitro ;
- R₇ représente un atome d'hydrogène, un groupe hydroxyle, ou un groupe alkyle ou hydroxyalkyle de 1 à 3 atomes de carbone ;
- R₈ représente un atome d'oxygène ou un groupe -NR₉R₁₀, R₉ et R₁₀, identiques ou différents, représentant un atome d'hydrogène, un groupe hydroxyle, ou un groupe alkyle ou hydroxyalkyle de 1 à 3 atomes de carbone, sous réserve que lorsque R₈ représente un atome d'oxygène alors a vaut 1, b représente une simple liaison et c représente une double liaison et que lorsque R₈ représente un groupe -NR₉R₁₀ alors a vaut 0, b représente une double liaison et c représente une simple liaison ;
ou sel pharmaceutiquement acceptable de celui-ci,
pour son utilisation dans la prévention ou le traitement de la maladie d'Alzheimer chez un individu par inhibition de l'action du peptide β-amyloïde.

2. Composé ou sel pharmaceutiquement acceptable de celui-ci pour son utilisation selon la revendication 1, dans lequel la formule (I) est représentée par la formule (II) suivante : 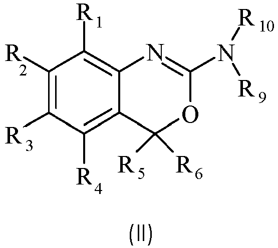 dans laquelle R₁, R₂, R₃, R₄, R₅, R₆, R₉ et R₁₀ sont tels que définis ci-dessus.

3. Composé ou sel pharmaceutiquement acceptable de celui-ci pour son utilisation selon la revendication 1 ou 2, de formule (IIIa), (IIIb) ou (IV) suivante :

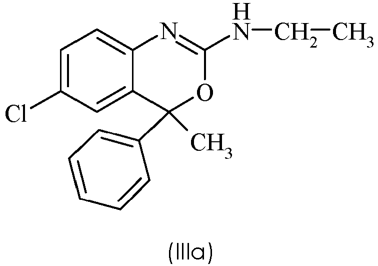

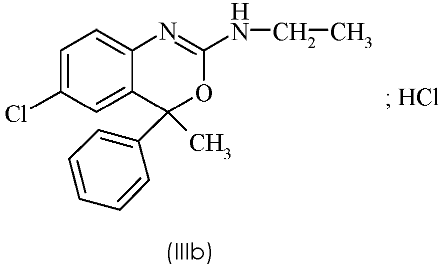

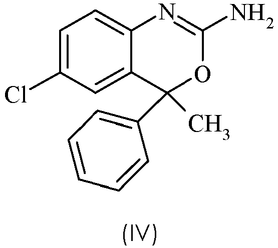

4. Composé ou sel pharmaceutiquement acceptable de celui-ci pour son utilisation selon la revendication 1, dans lequel la formule (I) est représentée par la formule (V) suivante : 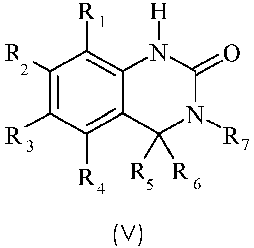 dans laquelle R₁, R₂, R₃, R₄, R₅, R₆, et R₇ sont tels que définis dans la revendication 1.

5. Composé ou sel pharmaceutiquement acceptable de celui-ci pour son utilisation selon la revendication 1 ou 4, de formule (VI) ou (VII) suivante :

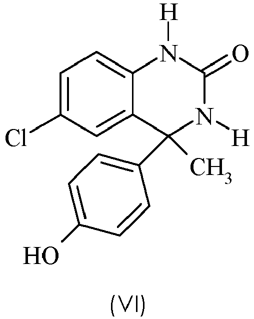

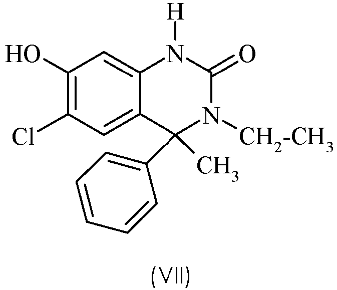

6. Composé ou sel pharmaceutiquement acceptable de celui-ci pour son utilisation selon l'une des revendications 1 à 5, conditionné en une dose unitaire de 50 mg à 1500 mg.

7. Composé ou sel pharmaceutiquement acceptable de celui-ci pour son utilisation selon l'une des revendications 1 à 6, sous une forme convenant pour une administration par voie orale.

8. Composé ou sel pharmaceutiquement acceptable de celui-ci pour son utilisation selon l'une des revendications 1 à 7, sous forme d'une poudre, de cachets, de gélules ou de sachets.

9. Composé ou sel pharmaceutiquement acceptable de celui-ci pour son utilisation selon l'une des revendications 1 à 8, en combinaison avec au moins un autre composé utile pour la prévention ou le traitement de la maladie d'Alzheimer.

10. Composé ou sel pharmaceutiquement acceptable de celui-ci pour son utilisation selon l'une des revendications 1 à 9, en combinaison avec au moins un autre composé utile pour la prévention ou le traitement de la maladie d'Alzheimer sélectionné dans le groupe constitué du donepezil, de la galantamine, de la memantine, et de la rivastigmine.

11. Composé ou sel pharmaceutiquement acceptable de celui-ci pour son utilisation selon l'une des revendications 1 à 10, dans laquelle l'individu présente des troubles cognitifs légers.

12. Composition pharmaceutique, comprenant à titre de substance active, au moins un composé de formule (I) tel que défini dans l'une des revendications 1 à 5, ou un sel pharmaceutiquement acceptable de celui-ci, éventuellement en association avec un véhicule pharmaceutiquement acceptable, et comprenant optionnellement au moins un autre composé utile pour la prévention ou le traitement de la maladie d'Alzheimer, notamment sélectionné dans le groupe constitué du donepezil, de la galantamine, de la memantine, et de la rivastigmine, pour son utilisation dans la prévention ou le traitement de la maladie d'Alzheimer par inhibition de l'action du peptide β-amyloïde, chez un individu, notamment un individu présentant des troubles cognitifs légers.

13. Produits contenant :
- au moins un composé de formule (I) tel que défini dans l'une des revendications 1 à 5, ou un sel pharmaceutiquement acceptable de celui-ci, et
- au moins un autre composé utile pour la prévention ou le traitement de la maladie d'Alzheimer, notamment sélectionné dans le groupe constitué du donepezil, de la galantamine, de la memantine, et de la rivastigmine,
comme produit de combinaison pour une utilisation dans la prévention ou le traitement de la maladie d'Alzheimer par inhibition de l'action du peptide β-amyloïde, chez un individu, notamment un individu présentant des troubles cognitifs légers.

## Patentansprüche

1. Verbindung der folgenden Formel (I): 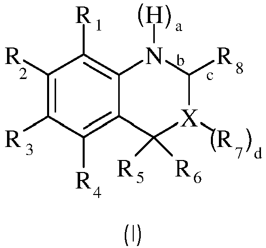 in der
- a für 0 oder 1 steht,
- b für eine Einfachbindung oder eine Doppelbindung,
- c für eine Einfachbindung oder eine Doppelbindung,
- d für 0 oder 1,
- X für ein Sauerstoff- oder Stickstoffatom, wobei in dem Fall, dass X für ein Sauerstoffatom steht, d den Wert 0 hat, und wenn X für ein Stickstoffatom steht, d den Wert 1 hat,
- R₁, R₂, R₃ und R₄, identisch oder unterschiedlich, für ein Wasserstoffatom, ein Halogenatom, insbesondere unter F, Cl, Br und I ausgewählt, eine Hydroxylgruppe oder eine Alkoxylgruppe mit 1 oder 2 Kohlenstoffatomen stehen,
- R₅ und R₆, identisch oder unterschiedlich, für ein Wasserstoffatom, eine Alkyl- oder Zykloalkylgruppe mit 1 bis 6 Kohlenstoffatomen oder eine Arylgruppe mit 6 Kohlenstoffatomen, deren aromatischer Kern gegebenenfalls mit einem oder mehreren Halogenatomen oder einer oder mehreren Hydroxylgruppen, Alkoxylgruppen mit 1 oder zwei Kohlenstoffatomen, Trifluormethyl- oder Nitrogruppen substituiert ist, stehen,
- R₇ für ein Wasserstoffatom, eine Hydroxylgruppe oder eine Alkyl- oder Hydroxyalkylgruppe mit 1 bis 3 Kohlenstoffatomen steht,
- R₈ für ein Sauerstoffatom oder eine Gruppe -NR₉R₁₀ steht, in der R₉ und R₁₀, identisch oder unterschiedlich, für ein Wasserstoffatom stehen, eine Hydroxylgruppe oder eine Alkyl- oder Hydroxyalkylgruppe mit 1 bis 3 Kohlenstoffatomen, wobei in dem Fall, dass R₈ für ein Sauerstoffatom steht, a den Wert 1 hat, b für eine Einfachbindung steht und c für eine Doppelbindung, und wenn R₈ für eine Gruppe -NR₉R₁₀ steht, a den Wert 0 hat, b für eine Doppelbindung steht und c für eine Einfachbindung,
oder ein pharmazeutisch verträgliches Salz davon,
zum Gebrauch in der Vorbeugung oder Behandlung der Alzheimer-Krankheit bei einem Patienten durch Inhibition der Wirkung des Peptids β-Amyloid.

2. Verbindung oder pharmazeutisch verträgliches Salz davon zum Gebrauch nach Patentanspruch 1, in der die Formel (I) die Form der folgenden Formel (II) hat 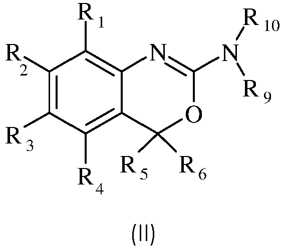 in der R₁, R₂, R₃, R₄, R₅, R₆, R₉ und R₁₀ wie oben definiert sind.

3. Verbindung oder pharmazeutisch verträgliches Salz davon zum Gebrauch nach Patentanspruch 1 oder 2 mit der folgenden Formel (IIIa), (IIIb) oder (IV):

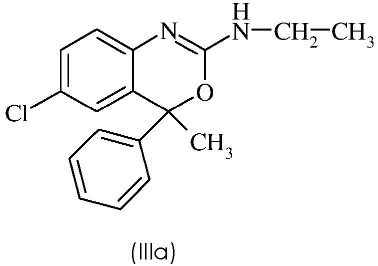

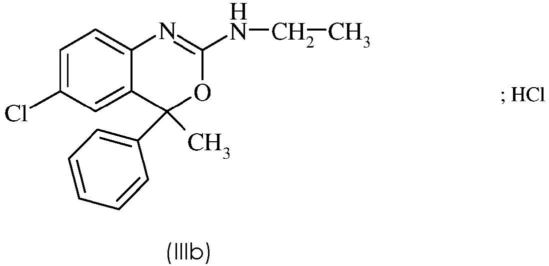

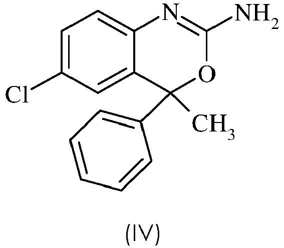

4. Verbindung oder pharmazeutisch verträgliches Salz davon zum Gebrauch nach Patentanspruch 1, in der die Formel (I) die Form der folgenden Formel (V) hat 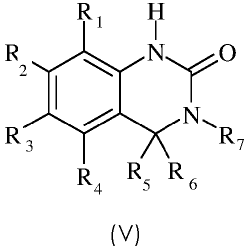 in der R₁, R₂, R₃, R₄, R₅, R₆, und R₇ wie in Patentanspruch 1 definiert sind.

5. Verbindung oder pharmazeutisch verträgliches Salz davon zum Gebrauch nach Patentanspruch 1 oder 4 mit der folgenden Formel (VI) oder (VII):

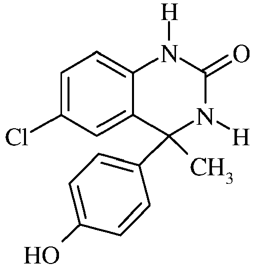

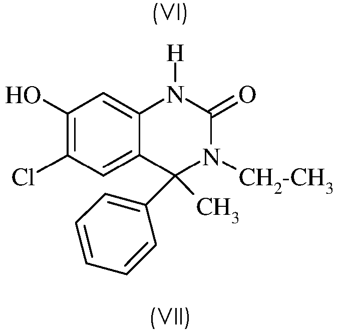

6. Verbindung oder pharmazeutisch verträgliches Salz davon zum Gebrauch nach einem der Patentansprüche 1 bis 5, in einer Einheitsdosis von 50 mg bis 1500 mg verpackt.

7. Verbindung oder pharmazeutisch verträgliches Salz davon zum Gebrauch nach einem der Patentansprüche 1 bis 6 in einer zur oralen Verabreichung geeigneten Form.

8. Verbindung oder pharmazeutisch verträgliches Salz davon zum Gebrauch nach einem der Patentansprüche 1 bis 7 in Form von Pulver, Tabletten, Gelkapseln oder Tüten.

9. Verbindung oder pharmazeutisch verträgliches Salz davon zum Gebrauch nach einem der Patentansprüche 1 bis 8 in Kombination mit mindestens einer anderen, zur Vorbeugung oder Behandlung der Alzheimer-Krankheit geeigneten Verbindung.

10. Verbindung oder pharmazeutisch verträgliches Salz davon zum Gebrauch nach einem der Patentansprüche 1 bis 9 in Kombination mit mindestens einer anderen, zur Vorbeugung oder Behandlung der Alzheimer-Krankheit geeigneten Verbindung, gewählt aus der Gruppe, bestehend aus Donepezil, Galantamin, Memantin und Rivastigmin.

11. Verbindung oder pharmazeutisch verträgliches Salz davon zum Gebrauch nach einem der Patentansprüche 1 bis 10, wobei der Patient leichte kognitive Störungen aufweist.

12. Pharmazeutische Zubereitung, die als Wirkstoff mindestens eine Verbindung der Formel (I) nach einem der Patentansprüche 1 bis 5 oder ein pharmazeutisch verträgliches Salz davon enthält, gegebenenfalls in Verbindung mit einem pharmazeutisch verträglichen Vehikel und optional mindestens eine andere, zur Vorbeugung oder Behandlung der Alzheimer-Krankheit geeignete Verbindung, insbesondere gewählt aus der Gruppe, bestehend aus Donepezil, Galantamin, Memantin und Rivastigmin, enthaltend, zum Gebrauch in der Vorbeugung oder Behandlung der Alzheimer-Krankheit bei einem Patienten durch Inhibition der Wirkung des Peptids β-Amyloid, insbesondere bei einem Patienten, der leichte kognitive Störungen aufweist.

13. Produkt umfassend:
- mindestens eine Verbindung der Formel (I) nach einem der Patentansprüche 1 bis 5 oder ein pharmazeutisch verträgliches Salz davon, und
- mindestens eine andere, zur Vorbeugung oder Behandlung der Alzheimer-Krankheit geeignete Verbindung, insbesondere gewählt aus der Gruppe, bestehend aus Donepezil, Galantamin, Memantin und Rivastigmin,
als Kombinationsprodukt zum Gebrauch in der Vorbeugung oder Behandlung der Alzheimer-Krankheit bei einem Patienten durch Inhibition der Wirkung des Peptids β-Amyloid, insbesondere einem Patienten, der leichte kognitive Störungen aufweist.

## Claims

1. A compound of the following formula (I): 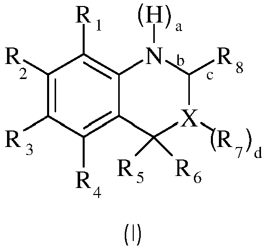 wherein:
- a represents 0 or 1;
- b represents a single bond or a double bond;
- c represents a single bond or a double bond;
- d represents 0 or 1;
- X represents an oxygen or nitrogen atom, with the proviso that when X represents an oxygen atom then d is 0 and when X represents a nitrogen atom then d is 1;
- R₁, R₂, R₃ and R₄, which may be identical or different, represent a hydrogen atom, a halogen atom, in particular selected from F, Cl, Br or I, a hydroxyl group, or an alkoxyl group of 1 or 2 carbon atoms;
- R₅ and R₆, which may be identical or different, represent a hydrogen atom, an alkyl or cycloalkyl group having 1 to 6 carbon atoms, or an aryl group having 6 carbon atoms, the aromatic ring of which is optionally substituted by one or more halogen atoms or one or more hydroxyl, alkoxyl having 1 or 2 carbon atoms, trifluoromethyl or nitro groups;
- R₇ represents a hydrogen atom, a hydroxyl group, or an alkyl or hydroxyalkyl group of 1 to 3 carbon atoms;
- R₈ represents an oxygen atom or an -NR₉R₁₀ group, R₉ and R₁₀, which may be identical or different, representing a hydrogen atom, a hydroxyl group, or an alkyl or hydroxyalkyl group of 1 to 3 carbon atoms, with the proviso that when R₈ represents an oxygen atom then a is 1, b represents a single bond and c represents a double bond and that when R₈ represents an -NR₉R₁₀ group then a is 0, b represents a double bond and c represents a single bond;
or a pharmaceutically acceptable salt thereof,
for use in the prevention or treatment of Alzheimer's disease in an individual by inhibiting the action of β-amyloid peptide.

2. The compound or pharmaceutically acceptable salt thereof for use according to claim 1, wherein formula (I) is represented by the following formula (II): 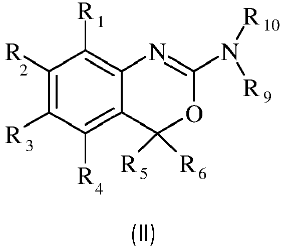 wherein R₁, R₂, R₃, R₄, R₅, R₆, R₉ et R₁₀ are as defined above.

3. The compound or pharmaceutically acceptable salt thereof for use according to claim 1 or 2, of the following formula (IIIa), (IIIb) or (IV):

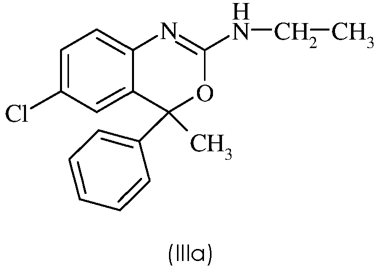

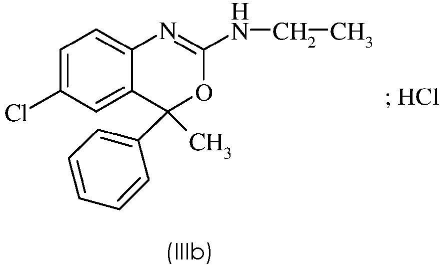

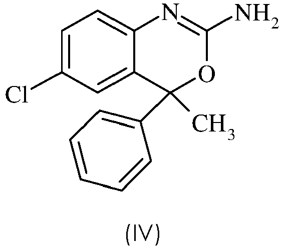

4. The compound or pharmaceutically acceptable salt thereof for use according to claim 1, wherein formula (I) is represented by the following formula (V): 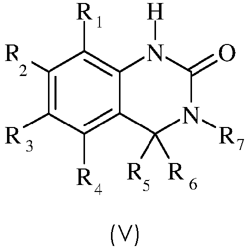 wherein R₁, R₂, R₃, R₄, R₅, R₆, et R₇ are as defined in claim 1.

5. The compound or pharmaceutically acceptable salt thereof for use according to claim 1 or 4, of the following formula (VI) or (VII):

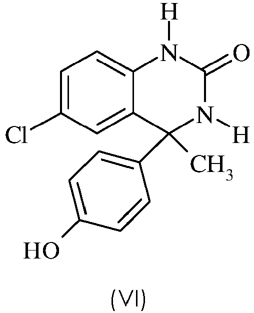

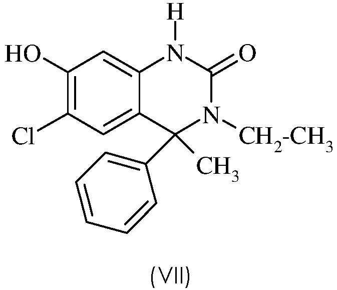

6. The compound or pharmaceutically acceptable salt thereof for use according to any one of claims 1 to 5, packaged in a unit dose of from 50 mg to 1500 mg.

7. The compound or pharmaceutically acceptable salt thereof for use according to any one of claims 1 to 6, in a form suitable for oral administration.

8. The compound or pharmaceutically acceptable salt thereof for use according to any one of claims 1 to 7, in the form of a powder, tablets, capsules or sachets.

9. The compound or a pharmaceutically acceptable salt thereof for use according to any one of claims 1 to 8, in combination with at least one other compound useful for the prevention or treatment of Alzheimer's disease.

10. The compound or a pharmaceutically acceptable salt thereof for use according to any one of claims 1 to 9, in combination with at least one other compound useful for the prevention or treatment of Alzheimer's disease selected from the group consisting of donepezil, galantamine, memantine, and rivastigmine.

11. The compound or pharmaceutically acceptable salt thereof for use according to any one of claims 1 to 10, wherein the individual has mild cognitive impairment.

12. A pharmaceutical composition, comprising as active substance at least one compound of formula (I) as defined in any one of claims 1 to 5, or a pharmaceutically acceptable salt thereof, optionally in combination with a pharmaceutically acceptable carrier, and optionally comprising at least one other compound useful for the prevention or treatment of Alzheimer's disease, in particular selected from the group consisting of donepezil, galantamine, memantine and rivastigmine, for use in the prevention or treatment of Alzheimer's disease by inhibiting the action of β-amyloid peptide in an individual, in particular an individual with mild cognitive disorders.

13. Products containing:
- at least one compound of formula (I) as defined in any one of claims 1 to 5, or a pharmaceutically acceptable salt thereof, and
- at least one other compound useful for the prevention or treatment of Alzheimer's disease, in particular selected from the group consisting of donepezil, galantamine, memantine and rivastigmine,
as a combination product for use in the prevention or treatment of Alzheimer's disease by inhibiting the action of β-amyloid peptide in an individual, in particular an individual with mild cognitive impairment.
